(19) **Europäisches Patentamt**
**European Patent Office**
**Office européen des brevets**

(11) **EP 3 810 050 B1**

(12) **EUROPEAN PATENT SPECIFICATION**

(45) Date of publication and mention of the grant of the patent:
**29.03.2023 Bulletin 2023/13**

(21) Application number: **18746323.7**

(22) Date of filing: **20.06.2018**

(51) International Patent Classification (IPC):
***A61F 9/009*** *(2006.01)*

(52) Cooperative Patent Classification (CPC):
**A61F 9/009**

(86) International application number:
**PCT/SI2018/050020**

(87) International publication number:
**WO 2019/245462 (26.12.2019 Gazette 2019/52)**

(54) **AN ACOUSTIC DIVERTER FOR IMPROVED SAFETY DURING OPHTHALMIC LASER TREATMENTS**

AKUSTISCHER ABLENKER FÜR VERBESSERTE SICHERHEIT BEI OPHTHALMISCHEN LASERBEHANDLUNGEN

DÉFLECTEUR ACOUSTIQUE POUR UNE SÉCURITÉ AMÉLIORÉE PENDANT DES TRAITEMENTS OPHTALMIQUES AU LASER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.04.2021 Bulletin 2021/17**

(73) Proprietor: **Optotek d.o.o.**
**1000 Ljubljana (SI)**

(72) Inventors:
• **VRECKO, Andrej**
**1000 Ljubljana (SI)**

• **POZAR, Tomaz**
**1360 Vrhnika (SI)**
• **PETKOVSEK, Rok**
**1000 Ljubljana (SI)**
• **ORTHABER, Uros**
**2331 Pragersko (SI)**

(74) Representative: **Patentni Biro AF d.o.o.**
**Kotnikova 32, p.p. 2706**
**1001 Ljubljana (SI)**

(56) References cited:
**EP-A1- 2 648 663          CH-A5- 694 936**
**US-A1- 2009 051 872    US-A1- 2015 217 129**

**Description**

Field of the invention

The present invention belongs to the field of instruments for examination and treatment of human eyes. The invention relates to an acoustic diverter, which is used during ophthalmic laser treatments based on photodisruption, such as capsulotomy, iridotomy and vitreolysis. More particularly, the invention relates to a therapeutic acoustic diverter which provides safer ophthalmic treatment as it minimizes damaging effects of laser-induced acoustic waves in the treated eye.

Background of the invention and the technical problem

Many ophthalmic laser treatments are used daily for treating different medical conditions, such as secondary cataract, closed angle glaucoma, presence of floaters in the vitreous body etc... Some of these treatments, e.g., posterior capsulotomy, iridotomy and vitreolysis, rely on photodisruption effects in order to cut, remove or puncture different tissues inside the eye. Photodisruption is a type of light-tissue interaction, where rupturing of a tissue is achieved by laser-induced optical breakdown inside the eye. High energy delivered by a strongly focused laser beam is concentrated in a very small focal spot, which results in a desired therapeutic effect on the tissue.

Among the most common laser therapies in ophthalmology is the posterior capsulotomy. Around 5 million capsulotomies are performed every year worldwide. This is a type of eye surgery in which an incision is made into the opacified capsule of the crystalline eye lens in order to restore vision. During the treatment, a pulsed laser beam is focused in the vicinity of the lens capsule, thus producing optical breakdown, which leads to the rupturing of the tissue.

Many laser shots are needed to completely clear the central area of the capsule.

Another treatment, which is based on photodisruption effects, is the iridotomy. The iridotomy uses a focused laser beam to create a hole on the outer edge of the iris. This opening allows aqueous humor to flow between the anterior chamber and the posterior chamber. This opening may decrease pressure in the eye and usually prevents sudden buildup of pressure within the eye, which occurs during an episode of acute closed-angle glaucoma.

The most recent ophthalmic therapy, which relies on photodisruption, is the vitreolysis. Here, laser pulses are used to break down the collagen opacities and other substances suspended in the vitreous humor known as floaters. As a result, floaters are removed from the optical axis or are significantly reduced in size. Some studies report that the total number of shots per session can reach up to 1000 or more (https://www.eyeworld.org/article-retina-clinical-committee-responds-to--pearls-for-yag-vitreolysis-of-floaters-).

Although treatments, which involve photodisruption were thought to be relatively safe, accumulating evidence shows that severe side effects can occur in patients that underwent capsulotomy, iridotomy and especially vitreolysis. After vitreolysis, the following complications were observed: retinal tears with retinal detachment, holes and other injuries, cataract formation, glaucoma, capsule defects, scotoma, increased number of floaters, as well as bleeding (Lim, 2017, JAMA Ophthalmology, 135(9): 924-925; Hahn et al., 2017, JAMA Ophthalmol. doi:10.1001/jamaophthalmol.2017.2477; Koo et al., 2017, Br J Ophthalmol 101:709-711; O'Day et al., 2018, Clin Exp Ophthalmol. doi: 10.1111/ceo.13190). The ASCRS Retina Clinical Committee reports that there is a definite risk of retinal tears and detachment when using YAG laser treatment in the vitreous (source: https://www.eyeworld.org/article-retina-clinical-committee-responds-to--pearls-for-yag-vitreolysis-of-floaters-). Similar side effects have been reported for capsulotomy, complications such as torn retina, infection, detached retina, glaucoma, macula holes and bleeding were observed. Also, for iridotomy, serious side effects have been reported, such as clouding of the lens, double vision, increased pressure, swelling of the cornea and macular tears (Anderson et al., 2006, Arch Ophthalmol 124(11): 1658-60; Sar et al., 2015, J Glaucoma 24(23): e14-5).

In all of the above procedures, the mechanisms causing the reported side effects are relatively unknown. However, what all of the above described treatments have in common is that therapeutic effect is achieved by photodisruption. This type of light-tissue interaction is initiated by laser-induced optical breakdown, which is followed by the emission of a high-amplitude shock wave (mechanical compression) and the production of a cavitation bubble. The latter two mechanical effects are mostly responsible for rupturing of the tissue. In the posterior segment, this shock wave may result not only in the desired therapeutic effect, but also in damage to the adjacent lens and retinal structures (Lim, 2017, JAMA Ophthalmology, 135(9): 924-925).

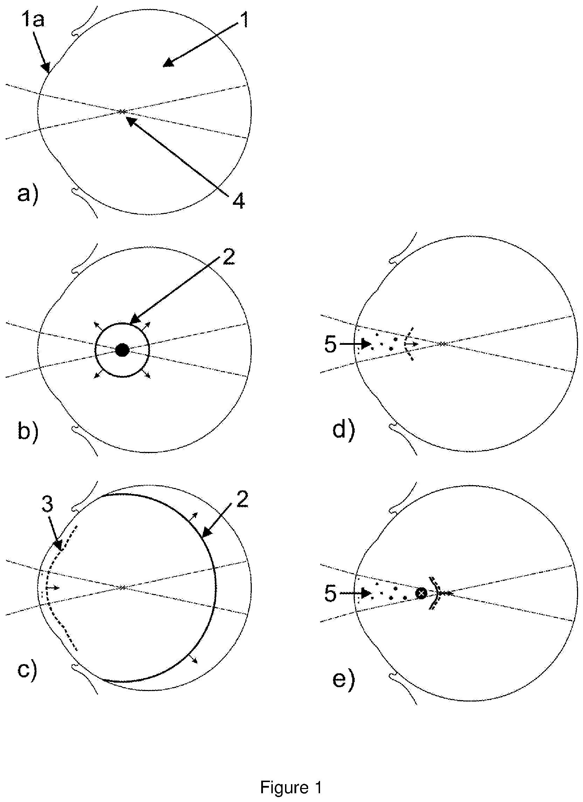

Mechanical effects of photodisruption, namely the cavitation bubble and the shock wave, represent a substantial threat for unwanted damage to intraocular structures. The cavitation bubble is formed in the optical breakdown region and is accompanied by an emission of a high-pressure mechanical wave, which initially propagates spherically away from the source with supersonic speed. These mechanical compressions in the form of elastic waves propagate through ocular tissues and are reflected and transmitted on each boundary between tissues with different acoustic impedances. Of considerable importance is the reflection of the compressional shock waves from the air-exposed surface of the cornea and sclera, which behaves as a concave (converging) mirror for acoustic waves (Figure 1). Upon reflection from the surface of the cornea and the sclera 1a of the human eye 1, the initial compression 2 is transformed into a high-amplitude rarefaction 3 (Figure 1c). If the optical breakdown 4 is performed near the optical axis, which is the case in most treatments, the reflected rarefaction is focused along the optical axis of the eye. In the focal volume, the maximum negative peak pressure of the rarefaction wave often surpasses -4 MPa, which corresponds to the tissue damage threshold (Coleman, 1993, Phys. Med. Biol., 38: 1545-1560). At negative peak pressure of -20 MPa, free field acoustic cavitation, a tissue-invasive mechanism, occurs in distilled water and soft tissues (T. Lee, J.G. Ok, L.J. Guo, and H.W. Baac, Appl. Phys. Lett. 108, 104102 (2016).). Rarefaction waves are especially critical, since negative-pressure amplitudes (already mentioned -4 MPa and -20 MPa thresholds) already cause unrepairable tissue damage at values much smaller than the positive-pressure amplitudes (80-100 MPa) (E.-A. Brujan and A. Vogel, J. Fluid Mech. 558, 281 (2006).

The effects of the rarefaction wave on the tissue are especially pronounced if a laser beam had just been transmitted through the same volume, since an illumination of the acoustic focal volume with laser light decreases the threshold for cavitation bubble formation. Such circumstances are typically encountered in laser eye treatments. Part of the laser beam energy is absorbed in the focal volume and forms microbubbles 5 which expand when the negative pressure pulse passes through that volume (Figure 1d in 1e). Even if the compression pulse is reflected at the cornea surface as a compression rather than rarefaction, as in the case when a contact treatment lens made of glass or similar material with high acoustic impedance is used, the compression still transforms into a rarefaction after focusing due to Gouy phase shift (Figure 1e). This rarefaction may also seriously threaten the retina as it propagates deeper into the vitreous body.

To illustrate the damage potential that acoustic waves represent, one can refer to different non-ophthalmic treatments, which use similar mechanical effects of high-intensity focused ultrasound (HIFU) such as destruction of cancerous formations, treatment of kidney stones, stimulation of bone growth and possibility of temporal interruption of blood-brain barrier to supply medications.

Since the pressure waves propagating within the eye represent significant risk to eye structures, it would be beneficial if their propagation and polarity inversion (transformation from compression to rarefaction) at the free boundaries was prevented. Thus, undesirable effects of the laser treatment would be reduced. However, there is currently no known device, which would prevent backfocusing of acoustic waves inside the eye. In order to reduce the risk for unwanted side effects during ophthalmic laser treatments, it would be desirable to provide an acoustic diverter for the use in capsulotomy, iridotomy, vitreolysis and other photodisruption-based ophthalmic treatments, so that undesired effects of acoustic waves (refocusing from the cornea or rarefaction generation) were prevented or at least limited.

The invention is defined in the claims.

State of the art

The hypothesis about the mechanisms underlying significant tissue injuries after eye treatments based on photodisruption is very recent, therefore there is no available prior art addressing the problem of how to prevent or reduce focusing of acoustic waves and their rarefaction in the eye.

Documents US2009/051872, CH694936 and US2015/217129 disclose conventional contact lenses used for laser treatments of the eye. Those contact lenses are typically made of glass or PMMA. Document EP2648663 discloses a iontophoresis apparatus adapted to be fixed on an eye and filled with a suitable substance.

Description of the invention

The invention will be described based on figures, which show:

Figure 1:     Acoustic wave propagation within the eye during ophthalmic surgery at approximately a) 0 μs, b) 3 μs, c) 9 μs, d) 11 μs and e) 14 μs after photoionization

Figure 2a:     Acoustic diverter according to the invention with a concave anterior surface

Figure 2b:     Acoustic diverter according to the invention with a flat anterior surface

Figure 2c:     Acoustic diverter according to the invention with a slightly convex anterior surface

Figure 3:     Acoustic wave propagation with the acoustic diverter according to the invention at approximately a) 0 μs, b) 3 μs, c) 8 μs, d) 10 μs and e) 13 μs after photoionization

Figure 4:     Acoustic wave propagation with a typical ophthalmic lens not suitable for reducing undesirable acoustic wave effects at approximately a) 0 μs, b) 3 μs, c) 8 μs, d) 14 μs and e) 18 μs after photoionization

Figure 5:     Acoustic diverter with additional entry lens

The present invention aims to provide an acoustic diverter for decreasing risk of damage to eye tissues, which is achieved with the system as defined in the independent claim. The preferred embodiments of the invention are defined in dependent claims. The acoustic diverter is manually placed onto the cornea of the eye during the laser treatment with a suitable laser for performing photodisruption based treatments. The task of the acoustic diverter is to prevent or at least minimize damaging effects of acoustic waves emitted during the ophthalmic laser treatments, particularly those based on photodisruption.

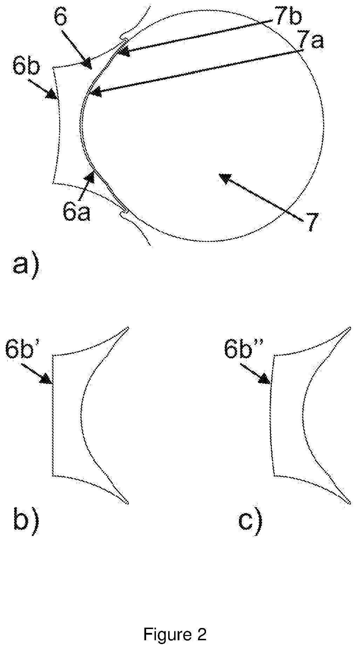

The present invention achieves the above discussed task by providing a system comprising a laser for performing photodisruption based treatments and an acoustic diverter, which has to have a geometry such that it prevents or decreases refocusing of acoustic waves back into the eye. The diverter is designed as a one-piece body having a surface to contact the eye and a surface away from the eye as shown in Figures 2a-2c. The surface of the acoustic diverter, which is in contact with the cornea of the eye is called the posterior surface. Conversely, the surface away from the eye is called the anterior surface. The geometry, more precisely the shape of the anterior surface of the acoustic diverter, influences whether the wave will be focused or dispersed.

The geometry of the acoustic diverter has to be such that the posterior surface is concave in order to adapt to average cornea curvature and the anterior surface is concave, flat or slightly convex (with a radius of more than 25 mm) in order to disperse the acoustic waves, so that focusing does not occur within the eye. Using such acoustic diverter, the negative peak pressures during the propagation of the pressure waves never exceed the injury threshold, thus preventing or at least minimizing eye injuries. The preferred geometry is that the anterior surface is concave, as this geometry provides the best dispersion of waves.

It is desirable that the acoustic diverter covers as much of the air-exposed surface of the eye as possible. Specifically, the air-exposed part of the sclera and the entire cornea, in order to prevent excessive contact between the eye and air, because this interface is subjected to significant wave reflection due to the differences in acoustic impendences of air and eye structures. Namely, acoustic waves are almost fully reflected and polarity-inverted from compressions to rarefactions when reflection takes place at the tissue-air interface.

Figure 2a shows a preferred embodiment of the acoustic diverter 6 according to the present invention placed onto the human eye 7, in which the right surface of the diverter is posterior 6a and in contact with the cornea and sclera 7a, while the left surface of the diverter is anterior 6b. This embodiment has a concave anterior surface 6b. Figures 2b and 2c show possible embodiments of the acoustic diverter having the anterior surface 6b' and 6b" flat or slightly convex with radius more than 25 mm, respectively.

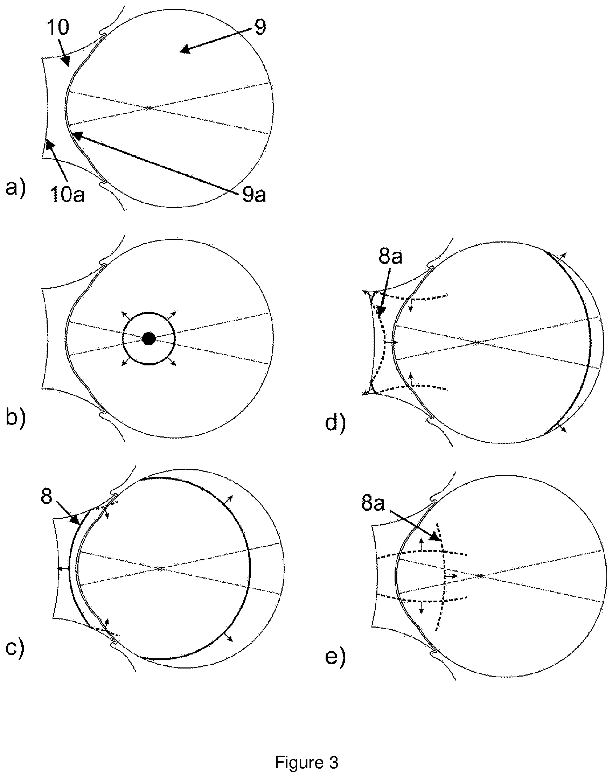

Figure 3 shows the propagation of acoustic waves when the acoustic diverter according to the invention is used. The compression waves 8, which are transmitted through the cornea 9a into the acoustic diverter 10 are reflected from the concave anterior surface 10a of the diverter back into the eye as diverging rarefaction 8a. Because of the divergent propagation, the rarefaction negative amplitude is not sufficient enough to cause damage to the eye structures after entering the eye 9.

The diverter may be made of any suitable material that has the following properties:

- transparency for the visible and near IR light,
- has to be autoclavable or sterilisable, and
- has to be biocompatible.

Such materials are glass, different plastics, polymethylmethacrylate (PMMA), polymethylpentene polymer (PMP), etc.

The properties of the material, namely its acoustic impedance $Z$, determine the reflected and transmitted amount of the acoustic wave at the diverter-cornea surface, and what is their pressure ratio with respect to the incoming wave. The reflection coefficient for the acoustic wave pressure amplitude on the interface between two materials can be calculated using the following formula:

$$r = (Z_1 - Z_2) / (Z_1 + Z_2)$$

in which r is the acoustic pressure reflection coefficient, $Z_1$ is the acoustic impedance of the material through which the wave initially propagates, $Z_2$ is the acoustic impedance of the material into which the wave is transmitted. In this case, $Z_1$ is related to the eye tissue, while $Z_2$ corresponds to the material of the acoustic diverter.

If the diverter is made of a material with acoustic impedance differing from the acoustic impedance of eye tissues (ranging from 1.4 to $1.7 \times 10^6$ kg/(m$^2$·s)) a part of the wave will be reflected. The acoustic impedance values for glass and PMMA are around $13 \times 10^6$ kg/(m$^2$·s) and $3.3 \times 10^6$ kg/(m$^2$·s), respectively. In case the material of the acoustic diverter is PMMA, the acoustic pressure reflection coefficient is r ≈ 35%, which means that the pressure amplitude of the reflected acoustic wave is 35% of the incident acoustic wave. In the case of glass, this reflection is approximately 80%. Due to the reflection from the contact surface between the eye and the acoustic diverter made of glass or PMMA, refocusing of the pressure wave within the eye still occurs. This means that both materials at least partly reduce the amplitude of the reflected acoustic waves, which reduces invasiveness of laser treatments based on photodisruption. If there is no element placed on the cornea, the amount of reflected wave will be almost 100%, since the acoustic impedance of air is $0.0004 \times 10^6$ kg/(m$^2$·s). Table 1 lists the acoustic pressure reflection coefficient for different materials used as an acoustic diverter.

Table 1: Amount of reflected acoustic wave for different materials

| Acoustic diverter material | Acoustic pressure reflection coefficient |
|---|---|
| No diverter (Air) | Almost 100% |
| Glass | 80% |
| PMMA | 35% |
| PMP | Close to 0% |

In order to ensure the transmission of acoustic waves without reflections, the acoustic diverter is made of a material with similar acoustic impedance as eye tissue, preferably the cornea. The human cornea has acoustic impedance of $1.7 \times 10^6$ kg/(m$^2$·s). When the material of the acoustic diverter features an acoustic impedance similar to the eye (between 1.4 and $1.7 \times 10^6$ kg/(m$^2$s)), the amount of reflected waves is almost 0. Hence, such material is the preferred candidate for acoustic diverter according to the invention, as focusing of acoustic waves will be prevented to a larger extent compared to when using glass or PMMA. A preferred example of a suitable material is polymethylpentene (PMP). PMP is a kind of a polyolefin and this expression covers different variants of the polymer, such as methylpentene homopolymer, or copolymers of 4-methylpentene-1 with olefinic monomers such as ethylene, propylene, butylene and higher olefins. It is commercially available from Mitsui Chemicals as TPX™-RT18. The acoustic impedance of PMP ranges from 1.46 to $1.70 \times 10^6$ kg/(m$^2$·s) at temperatures ranging from 25°C to 37°C, which is thus very close or equal to an acoustic impedance of the human eye tissues, making it particularly suitable for the acoustic diverter according to the invention. The synergistic effect of the acoustic diverter geometry and the material with acoustic impedance between 1.4 and $1.7 \times 10^6$ kg/(m$^2$·s) is that acoustic waves are not focused back into the eye but are effectively dispersed. Consequently, the negative peak pressures never exceed the threshold of -4 MPa, which means that eye injuries are prevented or minimized.

In addition to the geometry or to the geometry and the suitable acoustic impedance, large acoustic attenuation of the material is preferred, because in this case the waves are absorbed within the diverter and cannot be reflected from the anterior surface of the diverter towards the eye. It is thus desirable that the material used to manufacture the acoustic diverter has a large attenuation coefficient near the acoustic wave frequency of 10 MHz, which is the dominant frequency of shock waves encountered in ophthalmic surgeries. The attenuation coefficient should be preferably more than 1 cm$^{-1}$, which ensures that the majority of the wave is absorbed upon travelling the acoustic diverter back and forth. This way the effects of the reflected acoustic waves from the anterior surface of the diverter are further reduced.

According to the previous two paragraphs, in the ideal case, the acoustic diverter is made of the material which has an acoustic impedance in the range from 1.4 to $1.7 \times 10^6$ kg/(m$^2$·s) and attenuation coefficient of more than 1 cm$^{-1}$.

According to calculations, an acoustic diverter made of the material with acoustic impedance in the range from 1.4 to $1.7 \times 10^6$ kg/(m$^2$·s) and having an attenuation coefficient of more than 1 cm$^{-1}$ excluding the above-mentioned geometry of the anterior surface already ensures sufficient damping of the acoustic waves, because the acoustic waves would already have been sufficiently absorbed. Thus, the acoustic diverter with an arbitrary shape of the anterior surface (concave, convex or flat) made from the material with an acoustic impedance in the range from 1.4 to $1.7 \times 10^6$ kg/(m$^2$·s) and attenuation coefficient larger than 1 cm$^{-1}$ represents an alternative solution of the technical problem.

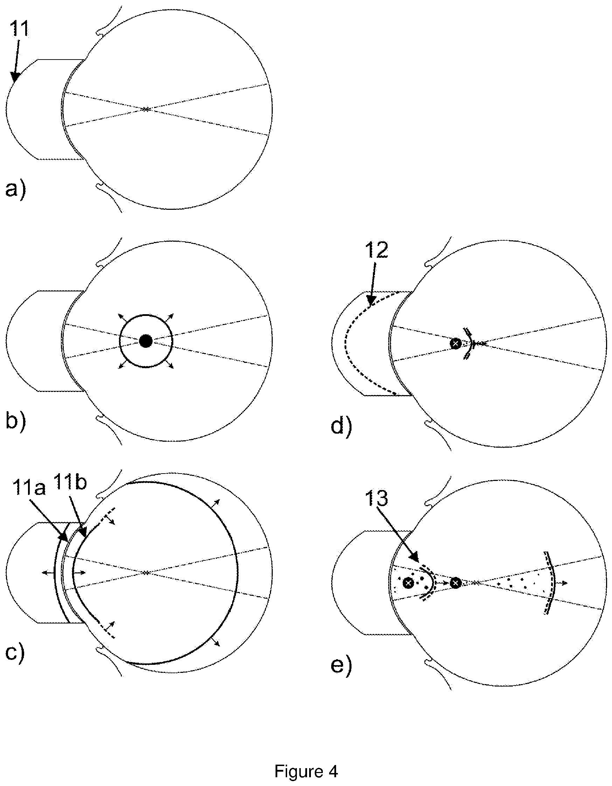

Usually, a physician uses a special contact ophthalmic lens while performing a treatment. Such ophthalmic lenses are specialized for a particular treatment, such as capsulotomy, iridotomy, vitreolysis. They are usually made of glass or PMMA material. However, they do not prevent the refocusing of the acoustic waves back into the eye due to the inappropriate geometry and material used as shown in Figure 4. A portion of the compression 11b is reflected already at the posterior surface 11a. The anterior surface 11 of these ophthalmic lenses is convex, which results in rarefaction focusing 12 back into the eye. Rarefaction focusing may also result in microbubble formation 13 in the laser illuminated parts of the eye. There exist some highly specialized lenses, like the ones according to WO2009094214 and CH694936A5, which can have non-convex anterior surface. However, they are not appropriate for any of the relevant photodisruption-based treatments, since they are all intended for diagnostics and treatment of peripheral regions of the eye and retina.

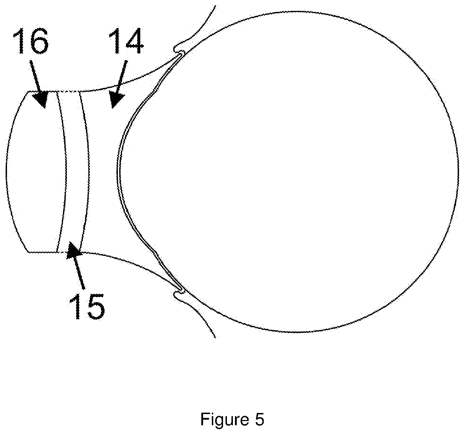

The present invention also provides a possibility to add to the acoustic diverter additional lenses in order to provide a collective system of lenses with an effective focal length most suitable for a particular treatment as shown in Figure 5. The acoustic diverter 14 and any possible additional lenses 16 may be made of the same material as the acoustic diverter described above or of a different material, provided that the material is suitable for making ophthalmology lenses, such as optical glass or polymeric material. Possible polymeric materials are PMMA, polycarbonate, polystyrene...

Preferably, the diverter 14 and any additional lenses 16 are made of a material having acoustic impedance 1.46 to $1.70 \times 10^6$ kg/(m$^2$·s), most preferably said elements are made of PMP. Additionally, the surfaces of the acoustic diverter and possible additional lenses may have an optical antireflection coating for visible and IR light. The most preferred embodiment has an optical antireflection coating on all surfaces.

In the embodiment, in which the acoustic diverter according to the invention is combined with an additional anterior lens, both parts are separated by air space 15. The separation between both elements is provided with any suitable distancing element, such as a distancing O-ring with the appropriate width or other suitable spacing elements in a well-known manner. There may be more than one lens following the acoustic diverter. In the embodiment with intermediate lenses, the above-mentioned air space is provided between the contact and the intermediate lens in the same way. The function of this air gap is to prevent any transmission of the acoustic waves through the anterior surface of the acoustic diverter to the additional lenses.

The system of the acoustic diverter and the laser is intended for the use in ophthalmology, in particular for the use in photodisruption laser treatments, such as capsulotomy, iridotomy and vitreolysis. The acoustic diverter according to the invention ensures that the acoustic waves are effectively dispersed so that they are not focused in the eye. Consequently, the negative peak pressures do not exceed the injury threshold, thus preventing or at least minimizing eye injuries. This is achieved by a specific geometry and/or specific material of the acoustic diverter.

**Claims**

1. A system consisting of:

   a laser adapted to perform photodisruption based laser treatments; and
   an acoustic diverter adapted for photodisruption based laser treatments for dispersing of acoustic waves to limit or prevent their focusing in the eye, which is designed as a one-piece body having a posterior surface for contacting the eye and an anterior surface, wherein the geometry of the anterior surface is concave, flat or slightly convex with a radius of curvature more than 25 mm, such that it prevents or decreases refocusing of acoustic waves back into the eye, and wherein the acoustic diverter is made of any material having the following properties:

   - transparency for the visible and near IR light,
   - has to be autoclavable or sterilisable, and
   - has to be biocompatible,

   **characterised in that** the material further has the following properties:

   - has acoustic impedance between 1.4 and $1.7 \times 10^6$ kg/(m$^2$·s) and
   - has acoustic attenuation coefficient more than 1 cm$^{-1}$ at 10 MHz.

2. The system according to claim 1, wherein the acoustic diverter is made of polymethylpentene (PMP).

3. The system according to claim 1 or claim 2, wherein the diverter has a concave anterior surface.

4. The system according to any of the preceding claims, wherein the diverter is adapted to cover the air-exposed part of the sclera and the entire cornea.

5. The system according to any of the preceding claims, wherein the diverter further includes one or more additional lenses to form a lens assembly, wherein the diverter and lenses are separated by air space.

6. The system according to any of the preceding claims, wherein the diverter includes an entry lens and optional intermediate lens, wherein the entry lens and optional intermediate lens is made of the same material as the acoustic diverter or any other suitable material.

7. The system according to any of the preceding claims, wherein all surfaces of the diverter have an optical coating for visible and IR light.

8. The system according to any claim from 1 to 7 wherein the laser is adapted to perform an iridotomy, capsulotomy or vitreolysis.

**Patentansprüche**

1. Ein System, bestehend aus:

   einem Laser zur Durchführung von Laserbehandlungen auf Basis von Photodisruption; und einem akustischen Ablenker für Laserbehandlungen auf Basis von Photodisruption zum Zerstreuen akustischer Wellen, um ihre Fokussierung im Auge zu begrenzen oder zu verhindern, der als einteiliger Körper mit einer hinteren Oberfläche für den Kontakt mit dem Auge und einer vorderen Oberfläche konstruiert ist, wobei die Geometrie der Vorderfläche konkav, flach oder leicht konvex mit einem Krümmungsradius von mehr als 25 mm ist, sodass er die Refokussierung akustischer Wellen zurück in das Auge verhindert oder verringert, und wobei der akustische Ablenker aus einem beliebigen Material hergestellt ist, das die folgenden Merkmale aufweist:

   - Transparenz für das sichtbare und nahe IR-Licht,
   - muss autoklavierbar oder sterilisierbar sein, und
   - muss biokompatibel sein,

   **dadurch gekennzeichnet, dass** das Material ferner die folgenden Merkmale aufweist:

   - eine akustische Impedanz zwischen 1,4 und $1,7 \times 10^6$ kg/(m$^2$·s), und
   - einen akustischen Dämpfungskoeffizienten von mehr als 1 cm$^{-1}$ bei 10 MHz.

2. System nach Anspruch 1, wobei der akustische Ablenker aus Polymethylpenten (PMP) gefertigt ist.

3. System nach Anspruch 1 oder Anspruch 2, wobei der Ablenker eine konkave vordere Oberfläche aufweist.

4. System nach einem der vorstehenden Ansprüche, wobei der Ablenker so gestaltet ist, dass er den der Luft ausgesetzten Teil der Sklera und die gesamte Hornhaut abdeckt.

5. System nach einem der vorstehenden Ansprüche, wobei der Ablenker ferner eine oder mehrere zusätzliche Linsen umfasst, um eine Linsenanordnung zu bilden, wobei der Ablenker und die Linsen durch einen Luftraum getrennt sind.

6. System nach einem der vorstehenden Ansprüche, wobei der Ablenker eine Eintrittslinse und eine optionale Zwischenlinse umfasst, wobei die Eintrittslinse und die optionale Zwischenlinse aus dem gleichen Material wie der akustische Ablenker oder einem anderen geeigneten Material hergestellt sind.

7. System nach einem der vorstehenden Ansprüche, wobei alle Oberflächen des Ablenkers eine optische Beschichtung für sichtbares und IR-Licht aufweisen.

8. System nach einem der Ansprüche 1 bis 7, wobei der Laser derart konzipiert ist, um eine Iridotomie, Kapsulotomie oder Vitreolyse durchzuführen.


**Revendications**

1. Un système se composant de :

   un laser adapté à la réalisation de traitements au laser à base de photodisruption ; et un déflecteur acoustique adapté aux traitements au laser à base de photodisruption, pour la dispersion des ondes acoustiques afin de limiter ou prévenir leur concentration dans l'œil, qui est conçu comme une pièce unique ayant une surface postérieure pour le contact avec l'œil et une surface antérieure, où la géométrie de la surface antérieure est concave, plate ou légèrement convexe avec un rayon de courbure supérieur à 25 mm, de façon à prévenir ou diminuer le recentrage des ondes acoustiques sur l'œil, et où le déflecteur acoustique se compose de tout matériau ayant les propriétés suivantes :

   - transparence pour la lumière visible et proche IR,
   - doit être autoclavable ou stérilisable, et
   - doit être biocompatible,

se **caractérise par le fait que** le matériau a également les propriétés suivantes :

- a une impédance acoustique comprise entre 1,4 et 1,7 $\times 10^6$ kg/(m$^2\cdot$s) et
- a un coefficient d'atténuation acoustique de plus de 1 cm$^{-1}$ à 10 MHz.

2. Le système selon la revendication 1, dans lequel le déflecteur acoustique se compose de polyméthylpentène (PMP).

3. Le système selon la revendication 1 ou la revendication 2, dans lequel le déflecteur a une surface antérieure concave.

4. Le système selon l'une quelconque des revendications précédentes, dans lequel le déflecteur est adapté pour couvrir la partie exposée à l'air de la sclère et de la cornée entière.

5. Le système selon l'une quelconque des revendications précédentes, dans lequel le déflecteur comprend en outre une lentille supplémentaire ou davantage pour former un assemblage de lentilles, où le déflecteur et les lentilles sont séparés par une couche d'air.

6. Le système selon l'une quelconque des revendications précédentes, dans lequel le déflecteur comprend une lentille d'entrée et une lentille intermédiaire facultative, où la lentille d'entrée et la lentille intermédiaire facultative se composent du même matériau que le déflecteur acoustique ou de tout autre matériau approprié.

7. Le système selon l'une quelconque des revendications précédentes, dans lequel toutes les surfaces du déflecteur possèdent un revêtement optique pour la lumière visible et proche IR.

8. Le système selon l'une quelconque des revendications 1 à 7, dans lequel le laser est adapté pour pratiquer une iridotomie, une capsulotomie ou une vitréolyse.

Figure 1

Figure 2

Figure 3

Figure 4

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009051872 A
- CH 694936
- US 2015217129 A
- EP 2648663 A
- WO 2009094214 A
- CH 694936 A5

**Non-patent literature cited in the description**

- **LIM.** *JAMA Ophthalmology,* 2017, vol. 135 (9), 924-925
- **HAHN et al.** *JAMA Ophthalmol,* 2017
- **KOO et al.** *Br J Ophthalmol,* 2017, vol. 101, 709-711
- **O'DAY et al.** *Clin Exp Ophthalmol,* 2018
- **ANDERSON et al.** *Arch Ophthalmol,* 2006, vol. 124 (11), 1658-60
- **SAR et al.** *J Glaucoma,* 2015, vol. 24 (23), e14-5
- **COLEMAN.** *Phys. Med. Biol.,* 1993, vol. 38, 1545-1560
- **T. LEE ; J.G. OK ; L.J. GUO ; H.W. BAAC.** *Appl. Phys. Lett.,* 2016, vol. 108, 104102
- **E.-A. BRUJAN ; A. VOGEL.** *J. Fluid Mech.,* 2006, vol. 558, 281